# EUROPEAN PATENT APPLICATION

(11) **EP 2 065 354 A1**
(43) Date of publication of application: **03.06.2009**
(21) Application number: 07807353.3
(22) Date of filing: 14.09.2007
(51) Int. Cl.: C07B 61/00, B01J 19/24, C07C 67/343, C07C 69/716

(54) **REACTION APPARATUS, AND REACTION METHOD**

(30) Priority: 19.09.2006 JP 2006253218
(71) Applicant: Banyu Pharmaceutical Co., Ltd., Chiyoda-ku Tokyo 102-8667 (JP)
(72) Inventor: YONEDA, Noriyuki, Kumagaya-shi Saitama 360-0214 (JP); ODA, Yuichi, Kumagaya-shi Saitama 360-0214 (JP); NAKANISHI, Yasuo, Kumagaya-shi Saitama 360-0214 (JP)
(74) Representative: Ede, Eric
(86) International application number: PCT/JP2007/067949
(87) International publication number: WO 2008/035633

(57) **Abstract**

The present invention is directed at obtaining a high yield of a target substance and simultaneously securing high productivity.

A reaction apparatus 10 has: a main flow channel 12 having an inner diameter of 3 mm, in which a raw material M₁ flows; an introduction flow channel 14 in which a raw material M₂ that causes a chemical reaction with the raw material M₁ flows; and five branch introduction flow channels 16a to 16e which are branched from the introduction flow channel 14 and introduce the raw material M₂ to the main flow channel 12, at predetermined introduction points 12o to 12s in the main flow channel 12. Here, in the main flow channel 12, the flow channel lengths of the flow channels 12b to 12d between adjacent introduction points 12p to 12s are not longer than those of the flow channels 12a to 12c between the next previous adjacent introduction points 12o to 12r in a flow direction of the raw material M₁. At least one length of the flow channels 12b to 12d between the adjacent introduction points 12p to 12s is shorter than lengths of the flow channels 12a to 12c between previous adjacent introduction points 12o to 12r in the flow direction of the raw material M₁.

## Description

### Technical Field

The present invention relates to a reaction apparatus which brings two types of fluids into a chemical reaction with each other and a reaction method therefor.

### Background Art

A conventional reaction apparatus has been proposed which brings two types of fluids into a chemical reaction with each other in a flow channel having a fine cross-section area so as to bring the two types of fluids into an efficient chemical reaction (see the following Patent document 1, for instance). This reaction apparatus can increase its specific surface (surface area per unit volume) of a reacting substance in a flow channel to be a reacting channel, in comparison with a conventional reaction method in a batch process, and accordingly can obtain high heat-removal efficiency. Thereby, the reaction apparatus can precisely control a reaction temperature, can realize a reaction under an ideal condition, and can realize an efficient reaction and a high yield. Particularly, when being applied to a reactant which causes a large exothermic reaction, the reaction apparatus can enhance a high yield. In recent years, an example according to the technology is described in which the apparatus uses a pipe having the inner diameter of 2 mm for a static mixer and has almost such a practical scale as to correspond to a volume of a production of 500 ml/min (see the following Patent document 2, for instance).
[Patent document 1]: Japanese Patent Laid-Open No. 2002-292271
[Patent document 2]: National Publication of International Patent Application No. 2003-523960

### Disclosure of the Invention

### Problem to be Solved by the Invention

A reaction apparatus having a diameter of a flow channel of approximately several hundreds µm (hereinafter referred to as microreactor) has been actively studied so far. However, when considering an industrial application, the reaction apparatus having the flow channel with a diameter of the above described size causes a problem of a plug-up in the flow channel due to a small dirt which has entered in a fluid and a crystal which has been formed through a fluid reaction. Particularly, some nucleophilic organometallic compound such as a Grignard reagent precipitates in the vicinity of 0°C. Then, the Grignard reagent approaches a temperature of a refrigerant in the flow channel because the microreactor effectively cools the Grignard reagent, and a speed in the flow channel is low, so that the plug-up of the flow channel tends to occur due to the precipitate. In addition, a temperature of the precipitation temperature or lower cannot be employed because of such a problem, which has been disadvantageous in a yield of a target substance.

In addition, it is difficult for the microreactor to make a fluid of a reacting substance flow at a larger flow rate than 20 ml/min. The microreactor also uses a pipe with a small diameter. From these reasons, it is difficult for the microreactor to secure sufficiently high productivity.

On the other hand, it is considered to simply increase the diameter to several millimeters. The method solves the problem of plug-up of the flow channel, but hardly provides the same heat removal efficiency as in the microreactor, and accordingly decreases the yield.

The present invention is designed at solving the above described problems, and is directed at providing a reaction apparatus which can provide a high yield of a target substance and secure high productivity, and providing a reaction method therefor.

### Means for Solving the Problem

A reaction apparatus according to the present invention having a main flow channel which has a cross-section area corresponding to an area of a circle having a diameter of 0.5 to 6 mm and in which a first fluid flows, an introduction flow channel in which a second fluid that causes a chemical reaction with the first fluid flows, and three or more branch introduction flow channels which are branched from the introduction flow channel and introduce the second fluid to the main flow channel at predetermined introduction points in the main flow channel is **characterized in that** a flow channel length between adjacent introduction points in the main flow channel is not longer than a flow channel length between next previous adjacent introduction points in a flow direction of the first fluid, and at least one flow channel length between the adjacent introduction points is shorter than flow channel lengths between previous adjacent introduction points in the flow direction of the first fluid.

In the reaction apparatus according to the present invention, the second fluid is introduced into the main flow channel at three or more introduction points of the main flow channel, and accordingly the reaction between the first fluid and the second fluid can proceed step by step. Thereby, the reaction apparatus can inhibit the temperature at one introduction point from rising due to the introduction of the second fluid. In the reaction apparatus according to the present invention, a temperature rise at an introduction point is lower as the introduction point is located downstream in a flow direction of the main flow channel, but the reaction apparatus has a structure in which a flow channel length between the adjacent introduction points in the flow direction of the first fluid is set so as not to be longer than a flow channel length between the next previous adjacent introduction points in the flow direction of the first fluid, and at least one flow channel length between the adjacent introduction points is shorter than a flow channel length between the previous adjacent introduction points in the flow direction of the first fluid, and accordingly can adequately remove the heat. From these reasons, the reaction apparatus according to the present invention can provide high heat-removal efficiency, and accordingly can provide a high yield of a target substance.

In a reaction system using a large tank, the temperature change needs to be reduced by diluting a compound with a solvent and increasing the heat capacity, in order to practically control the temperature rise due to a reaction heat. On the other hand, the reaction apparatus according to the present invention can control the temperature to a target temperature without diluting the compound with the solvent.

The reaction apparatus according to the present invention has a larger cross-section area of the flow channel than a microreactor, and thereby can prevent the plug-up of the flow channel and can secure high productivity.

The reaction apparatus desirably further has temperature control means for controlling the temperature of the main flow channel and the vicinity of the introduction point in the branch introduction flow channel. The reaction apparatus having such a structure can surely implement the present invention.

The cross-section area of the main flow channel is desirably equivalent to the area of a circle having a diameter of 1 to 3 mm. The reaction apparatus having such a structure can provide a more preferable result in at least any one of the yield and the productivity.

The introduction point is preferably structured by a 180-degree T-shaped mixture channel, and the branch introduction flow channel is perpendicularly connected to the main flow channel. The reaction apparatus having such a structure can easily implement the present invention, and can realize the space saving of the apparatus.

The number of the branch introduction flow channels is desirably 5 to 10. The reaction apparatus having such a structure can further distribute the temperature rise caused by a reaction between the first fluid and the second fluid, and can surely show an effect according to the present invention.

The reaction apparatus desirably has further a first adjusting flow channel for adjusting the temperature of the first fluid before the first fluid is supplied to the main flow channel, and a second adjusting flow channel for adjusting the temperature of the second fluid before the second fluid is supplied to the introduction flow channel. The reaction apparatus having such a structure can surely control the temperatures of the first fluid and the second fluid.

The main flow channel, the introduction flow channel and the branch introduction flow channel desirably have the same cross-section area, and the branch introduction flow channels desirably have the same flow channel length.

The main flow channel and the branch introduction flow channel have desirably the cross-section areas not larger than that of the introduction flow channel, and the branch introduction flow channels have desirably the same flow channel length

The main flow channel and the branch introduction flow channel have desirably cross-section areas not larger than that of the introduction flow channel, and the branch introduction flow channel has a flow channel length not shorter than that of a branch introduction flow channel to be connected to the main flow channel at the introduction point next previous to the introduction point of the branch introduction flow channel, in the flow direction of the main flow channel.

The reaction apparatus desirably has further a first pump which supplies the first fluid to the main flow channel, and a second pump which supplies the second fluid to the introduction flow channel. The reaction apparatus having such a structure can surely use the reaction apparatus according to the present invention.

The first pump and the second pump are desirably a double diaphragm pump which employs a non-circular cam therein. The reaction apparatus having such a structure can make a pulsating flow in the fluid small, can stably obtain a high yield due to an accurate flow rate, and can surely show the effect according to the present invention. Furthermore, the reaction apparatus can continuously supply a fluid having high reliability for a long period of time without making a fine solid such as dirt in the fluid caught in the pump.

By using the above described reaction apparatus, various reactions are enabled.

Specifically, a reaction method using the above described reaction apparatus can be implemented that passes a fluid containing a nucleophilic organometallic compound as one of the first fluid and the second fluid, and passes a fluid containing a compound which causes an addition reaction or an exchange reaction with the nucleophilic organometallic compound, as the other fluid.

Though the structure varies according to the characteristics of the reaction and a target compound to be obtained, a by-product of a sequential reactant tends to be formed by a further progressed synthesis reaction than a target compound, when the nucleophilic organometallic compound excessively exists with respect to a compound which causes the addition reaction or the exchange reaction with the nucleophilic organometallic compound. For this reason, it is preferable to pass the fluid containing the nucleophilic organometallic compound as the second fluid, and to pass the fluid containing the compound which causes the addition reaction or the exchange reaction with the nucleophilic organometallic compound as the first fluid. The reaction apparatus having such a structure can complete the reaction in the vicinity of the introduction point, does not increase the concentration of the nucleophilic organometallic compound in the reaction fluid, produces few by-products and can produce with high efficiency.

In addition, the reaction apparatus can sequentially and efficiently react a fluid which causes a chemical reaction with the obtained product. Specifically, the sequential reaction can be efficiently carried out through two continuous reaction apparatuses, by passing a fluid obtained as a product from the reaction apparatus through a reaction apparatus having the same structure, as any one of the first fluid and the second fluid, and passing a fluid which causes a chemical reaction with the obtained product as the other fluid.

In order to enhance the reactivity, the first fluid and the second fluid preferably contain at least one type of solvent selected from the group consisting of tetrahydrofuran, diethyl ether, dioxane and dibutyl ether.

In this case, it is possible to make a fluid containing the nucleophilic organometallic compound out of the first fluid and the second fluid contain at least one type of solvent selected from the group consisting of tetrahydrofuran, diethyl ether, dioxane and dibutyl ether, and make the fluid containing the compound which causes the addition reaction or the exchange reaction with the nucleophilic organometallic compound contain no solvent. The reaction apparatus having such a structure can provide a target substance having high concentration.

The nucleophilic organometallic compound shall preferably be at least one compound selected from the group consisting of an organomagnesium compound (Grignard reagent, in particular), an organolithium compound, an organozinc compound, an organocadmium compound and an organosodium compound. In addition, the compound which causes the addition reaction or the exchange reaction with the nucleophilic organometallic compound is preferably a carbonyl compound.

Such a nucleophilic organometallic compound has excellent reactivity with a reactive substrate such as a carbonyl compound, and can produce a target substance at a high yield. A specific example of a reaction with the use of the carbonyl compound includes a reaction between 1-bromomagnesium-5-chloropentane employed as the nucleophilic organometallic compound and diethyl oxalate employed as a compound which causes the addition reaction or the exchange reaction with the nucleophilic organometallic compound.

A reaction apparatus according to the present invention can also be applied to other reactions than the above described reactions. For instance, the reaction apparatus can be applied to a reaction method of passing a fluid containing a catalyzer for a reaction selected from hydrogenation or reduction and hydrogen as one of the first fluid and the second fluid, and passing a fluid containing a substrate for the reaction as the other.

In this case, it is preferable to pass a fluid containing the catalyzer which is made for a hydrogenation reaction from a metallic complex having ferroceno phosphine as a ligand, and hydrogen, as one of the first fluid and the second fluid, and to pass a fluid containing an unsaturated compound as the other. In this case, a rhodium complex having ferroceno phosphine as a ligand is particularly suitable for the metallic complex.

### Advantage of the Invention

According to the present invention, high heat removal efficiency can be obtained, and accordingly a high yield of a target substance can be obtained. A reaction apparatus according to the present invention has a larger cross-section area of a flow channel than a microreactor, and accordingly can prevent the plug-up of the flow channel and can secure the high productivity.

### Brief Description of the Drawings

Figure 1 is a block diagram of a reaction apparatus according to an embodiment of the present invention;
Figure 2 is another block diagram of a reaction apparatus according to an embodiment of the present invention;
Figure 3 is a view of a reaction apparatus according to an embodiment of the present invention, which is viewed from an upper part; and
Figure 4 is a view of a reaction apparatus according to an embodiment of the present invention, which is viewed from a side face.

### Denotation of Symbols

10 and 50 --- reaction apparatus,
12 --- main flow channel,
12a to 12e --- flow channel from each of introduction point of main flow channel to next introduction point,
12o to 12s --- introduction point,
14 --- introduction flow channel,
14o to 14s --- branch point,
16a to 16e --- branch introduction flow channel,
18 and 24 --- adjusting flow channel,
20 and 26 --- pump,
22 --- container,
28 --- thermostatic liquid tank,
30 --- refrigerant,
32--- flowing-type fine reaction flow channel,
34 --- temperature controller,
36 --- heat exchanger,
38 --- cooling pipe.

### Best Mode for Carrying Out the Invention

Preferred embodiments of a reaction apparatus according to the present invention will now be described below with reference to the drawings. In the description for the drawings, the same elements are identified by the same reference numerals, and overlapping descriptions are omitted.

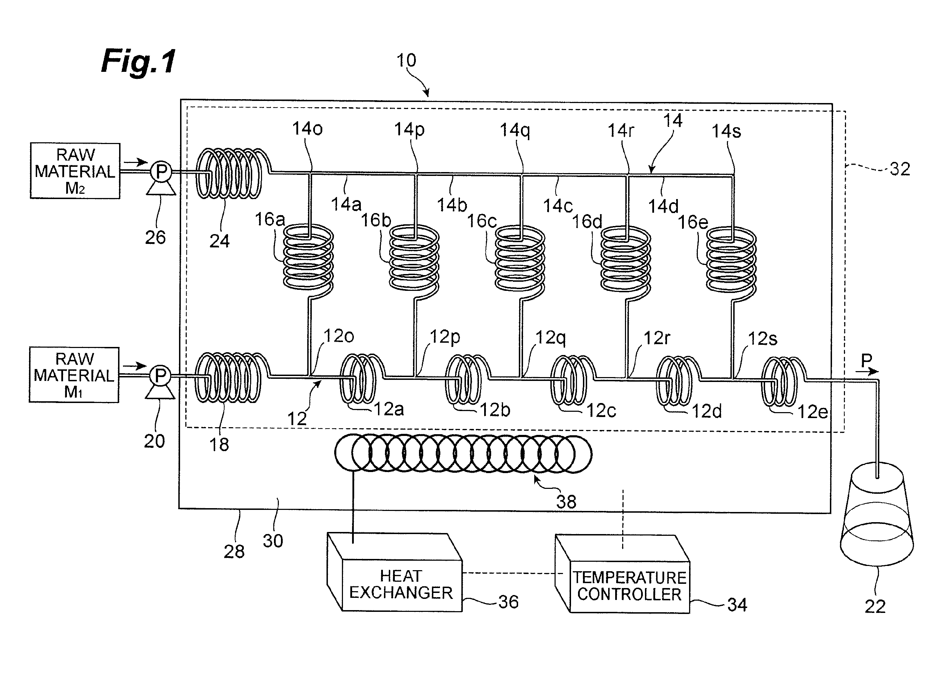

Figure 1 illustrates a reaction apparatus 10 according to the present embodiment. The reaction apparatus 10 is an apparatus for producing a target substance by bringing raw materials M₁ and M₂ which are two types of fluids into a chemical reaction in a flow channel. The reaction apparatus 10 has a main flow channel 12 in which the raw material M₁ of a first fluid flows, and an introduction flow channel 14 in which the raw material M₂ of a second fluid flows. The main flow channel 12 and the introduction flow channel 14 are composed of, for instance, a circular pipe which is made from stainless and has the inner diameter of 3 mm and the outer diameter of 4 mm.

Each of the flow channels 12 and 14 needs not to employ the circular pipe, but the flow channel 12 employs a pipe having the cross-section area equivalent to the area of a circle having a diameter (inner diameter) of 0.5 to 6 mm from the viewpoint of the productivity of the target substance and the like. That is to say, the equivalent diameter of the main flow channels 12 is set at 0.5 to 6 mm. In addition, the equivalent diameter is desirably set at 1 to 3 mm from at least any one viewpoint of the yield and the productivity.

The main flow channel 12 and the introduction flow channel 14 are connected to each other through a plurality of branch introduction flow channels 16a to 16e. The branch introduction flow channels 16a to 16e are branched at branch points 14o to 14s from the introduction flow channel 14 respectively, are connected to the main flow channel 12 at predetermined introduction points 12o to 12s in the main flow channel 12, and introduce the material M₂ flowing in the introduction flow channel 14 into the main flow channel 12. The branch introduction flow channels 16a to 16e are perpendicularly connected to the introduction flow channel 14 at the branch points 14o to 14s. Specifically, the branch points 14o to 14s is composed of, for instance, a 180-degree T-shaped mixture flow channel made from stainless steel, and can employ, for instance, a T-shaped joint made by Swagelok Company.

The material M₂ which has been introduced into the main flow channel 12 through the branch introduction flow channels 16a to 16e reacts with the material M₁ which flows in the main flow channel 12 to produce a target substance (or substance for producing the target substance). The branch introduction flow channels 16a to 16e are composed of, for instance, a circular pipe which is made from stainless steel and has the inner diameter of 1 mm and the outer diameter of 3 mm.

In addition, the branch introduction flow channels 16a to 16e are perpendicularly connected to the main flow channel 12 at the introduction points 12o to 12s. Specifically, the introduction points 12o to 12s are composed of, for instance, a 180-degree T-shaped mixture flow channel made from stainless steel (can employ a T-shaped joint made by Swagelok Company, for instance). Note that the pipe diameter and the length of the main flow channel 12, the introduction flow channel 14 and each of the branch introduction flow channels 16a to 16e are adjusted so that the flow rate of the material M₂ to be introduced into the main flow channel 12 from each of the branch introduction flow channels 16a to 16e can be approximately equal, or the flow rate can be slightly more than that of the branch introduction flow channels 16a to 16e to be connected to the main flow channel 12 downstream in a flow direction.

For instance, the main flow channel 12, the introduction flow channel 14 and the branch introduction flow channels 16a to 16e may have the same equivalent diameter (that is to say, the same cross-section area), and the branch introduction flow channels 16a to 16e may have the same flow channel length with each other. Furthermore, the main flow channel 12 and the branch introduction flow channels 16a to 16e may have an equivalent diameter not larger than the introduction flow channel 14 (specifically, the main flow channel 12 and the branch introduction flow channels 16a to 16e have cross-section areas not larger than the introduction flow channel 14), and the branch introduction flow channels 16a to 16e may have the same flow channel length with each other. More preferably, the main flow channel 12 and the branch introduction flow channels 16a to 16e have an equivalent diameter not larger than the introduction flow channel 14, and the branch introduction flow channels 16a to 16e may have the flow channel length not shorter than that of the respective branch introduction flow channels 16a to 16d which are connected to the main flow channel 12 at the respective next previous introduction points 12o to 12r of the branch introduction flow channels 16b to 16e in a flow direction of the main flow channel 12.

The branch introduction flow channels 16a to 16e may have equivalent diameters not larger than that of the introduction flow channel 14, and may be set at, for instance, 1 mm. When the flow rate of the branch introduction flow channels 16a to 16e is set at 100 ml/min or more, the branch introduction flow channels 16a to 16e may have the equivalent diameter of 3 mm.

In the main flow channel 12, the flow channels 12a to 12d from the respective introduction points 12o to 12r to next respective introduction points 12p to 12s and the flow channel 12e from the last introduction point 12s to an edge of a thermostatic liquid tank 28 may be formed into a coil shape for space saving. The branch introduction flow channels 16a to 16e may be similarly formed into a coil shape.

In order to realize a simple structure, flow channels 14a to 14d between the branch points 14o to 14s of the introduction flow channel 14 can have the same flow channel length. In this case, as the branch introduction flow channels 16a to 16e are branched downstream, the flow rate of a raw material M₂ which is passed as a branched flow increases. The flow channel lengths of the flow channels 14a to 14d between the branch points 14o to 14s of the introduction flow channel 14 shall be respectively equal to that of the flow channels 12a to 12d between the introduction points 12o to 12s of the main flow channel 12 corresponding to the branch points 14o to 14s. Specifically, the flow channel 14a of the introduction flow channel 14 has the same length as that of the flow channel 12a of the main flow channel 12, and the flow channels 14b to 14d of the introduction flow channel 14 also have their lengths similarly. The reaction apparatus having such a structure can pass an almost same amount of the raw material M₂ in the branch introduction flow channels 16a to 16e, and can provide a high yield, which is preferable. Preferably, the flow channel lengths of the branch introduction flow channels 16a to 16e are normally set at 0.5 m to 3.0 m, and can have the same length. Specifically, all lengths of each of the branch introduction flow channels 16a to 16e are set at 0.5 m, for instance. Furthermore, the lengths are preferably set at 0.5 m, 1.5 m, 2.0 m, 2.5 m and 3.0 m respectively.

In the main flow channel 12, the flow channel lengths between adjacent introduction points 12p to 12s in a flow direction of the raw material M₁ are not longer than the next previous flow channel length between adjacent introduction points 12o to 12r in the flow direction of the first fluid. In addition, at least one of the flow channel lengths between adjacent introduction points 12p to 12s is shorter than the flow channel length between the next previous adjacent introduction points 12o to 12r. Specifically, the flow channel length of the flow channel 12a between the introduction points 12o and 12p is set at 1 m, and the flow channel length of the flow channels 12b to 12d between the adjacent introduction points 12p to 12s other than the above flow channel length is set at 0.5 m, for instance. This is because the temperature rise in the introduction points 12o to 12s is lower in a more downstream flow direction of the raw material M₁ in the main flow channel 12 and accordingly the structure can appropriately remove the heat.

In the present embodiment, five branch introduction flow channels 16a to 16e are installed, but at least three branch introduction flow channels may be installed. The number of the branch introduction flow channels is desirably 5 to 10. This is because the temperature rise due to a chemical reaction between a raw material M₁ and a raw material M₂ is further distributed, and accordingly the structure can surely show an effect of the present invention.

A first adjusting flow channel 18 which adjusts a raw material M₁ to an optimum temperature beforehand is provided in an upstream direction of a first introduction point 12o of a main flow channel 12. Specifically, the adjusting flow channel 18 is integrally composed with a circular pipe which constitutes the main flow channel 12. A first pump 20 which supplies the raw material M₁ to the main flow channel 12 is provided in a further upstream direction of the circular pipe. A container 22 for collecting a produced liquid P which has been produced by a reaction between the raw material M₁ and the raw material M₂ is provided on the opposite end of the main flow channel 12.

A second adjusting flow channel 24 for adjusting the raw material M₂ to the optimum temperature beforehand is provided in an upstream direction (in a flow direction of raw material M₂) of a point at which the branch introduction flow channel 16a firstly branches off from an introduction flow channel 14. Specifically, the adjusting flow channel 24 is integrally composed with a circular pipe which constitutes the introduction flow channel 14. A second pump 26 which supplies the raw material M₂ to the introduction flow channel 14 is provided in a further upstream direction of the circular pipe. It is preferable to form the adjusting flow channels 18 and 24 into a coil shape for space saving, similarly to the main flow channel 12 and the branch introduction flow channels 16a to 16e.

The above described pumps 20 and 26 desirably employ a double diaphragm pump which employs a non-circular cam so as to make a pulsating current of a fluid to be supplied small. This pump can employ specifically a smooth flow pump TPL1M or TLP2M made by TACMINA CORPORATION, for instance.

The above described main flow channel 12, the introduction flow channel 14, the branch introduction flow channels 16a to 16e, and the adjusting flow channels 18 and 24 are arranged in a thermostatic liquid tank 28. A refrigerant 30 is contained in the thermostatic liquid tank 28, and cools fluids which flow through the main flow channel 12, the introduction flow channel 14, the branch introduction flow channels 16a to 16e and the adjusting flow channels 18 and 24 (which are generically referred to as flowing type fine reaction flow channel 32). The refrigerant 30 is kept at a constant temperature by a temperature controller 34, a heat exchanger 36 and a cooling pipe 38 which are provided on the thermostatic liquid tank 28. In other words, the thermostatic liquid tank 28, the refrigerant 30, the temperature controller 34, the heat exchanger 36 and the cooling pipe 38 are temperature control means for controlling the temperatures of the main flow channel 12 and the branch introduction flow channels 16a to 16e. However, the temperature control means does not necessarily need to have the above described structure, but may be any means as long as it appropriately controls the temperature. For instance, there is a method of keeping the liquid in the tank at a constant temperature by installing a cooler in the outside and directly circulating the refrigerant 30 in the cooler without using the cooling pipe.

The temperature control means is directed at controlling the temperature of a fluid which flows in the flow channel in the vicinity of the introduction points 12p to 12s, in the main flow channel 12 and the branch introduction flow channels 16a to 16e. One thermostatic liquid tank 28 may be provided for the flowing-type fine reaction flow channel 32 in order to simplify the apparatus, and a refrigerant to be supplied may be controlled to one temperature. Accordingly, the reaction heat to be removed is adjusted with the flow channel length or the time in which the fluid stays in the flow channel. That is to say, the temperature of the fluid in the main flow channel 12 can be brought close to a target temperature, by making at least one flow channel length between adjacent introduction points 12p to 12s in a flow direction of the M₁ shorter than that between the next previous introduction points in the main flow channel 12, in principle, as was described above. The flow channel lengths of each of the flow channels 12a to 12e are normally set at 0.5 m to 3.0 m, and as the flow rate increases, the flow channel length increases.

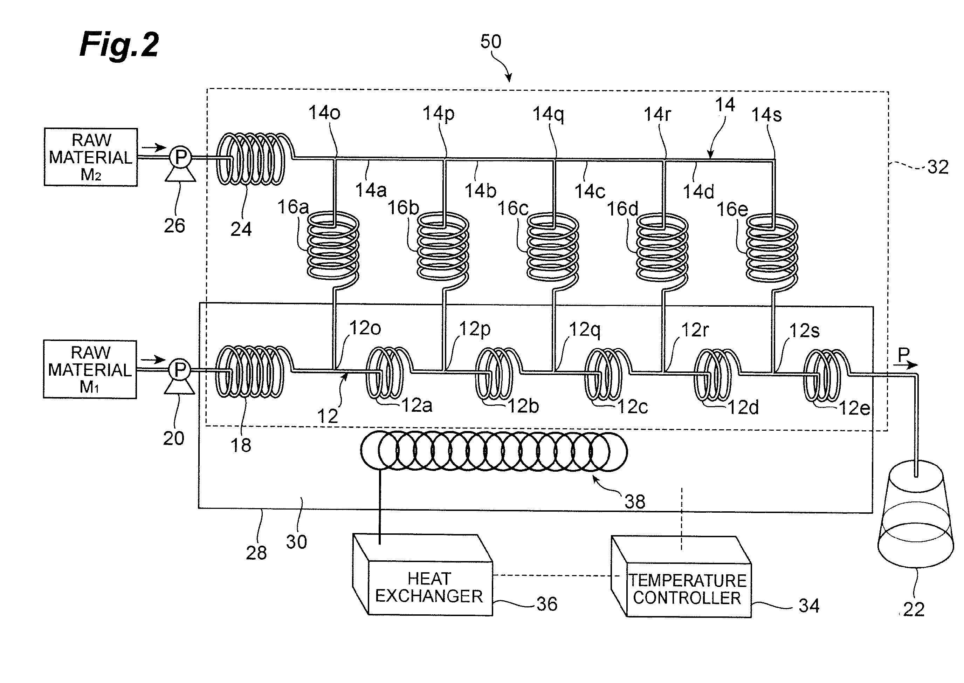

On the other hand, the temperature of the fluid to be supplied to the introduction points 12p to 12s from the branch introduction flow channels 16a to 16e may be controlled by the flow channel lengths of the branch introduction flow channels 16a to 16e to be arranged in the thermostatic liquid tank 28. As the temperature of the refrigerant is lower, the lengths of the flow channels to be arranged are shorter, and as the flow rates of the fluid to be passed to the branch introduction flow channels 16a to 16e are smaller, the lengths may be shorter. When a refrigerant at -15 to -30°C is used, the apparatus may normally have a structure as illustrated in Figure 2 that will be described later, in which the branch introduction flow channels 16a to 16e are arranged in the thermostatic liquid tank 28 by the depth of approximately 5 cm. This is because when the nucleophilic organometallic compound is passed in the branch introduction flow channels 16a to 16e, the nucleophilic organometallic compound stably continues to flow in the branch introduction flow channels for a long period of time without causing plug-up. In this case, the heat is mainly removed in the main flow channel 12. When the refrigerant at a temperature of -5 to -15°C or higher is used, it is efficient to arrange all branch introduction flow channels 16a to 16e in the thermostatic liquid tank 28, as is illustrated in Figure 1.

The temperature of the refrigerant 30 needs to be set at a low temperature, as the flow rate (= flow rate of introduction flow channel 14 + flow rate of main flow channel 12) is larger, and as the equivalent diameter of the main flow channel 12 is larger, so as to provide desirable cooling efficiency. Normally, when the equivalent diameter of the main flow channel 12 is 3 mm, the temperature is set at - 15 to -30°C, and when the equivalent diameter is 1 mm, the temperature is set at -5 to -15°C.

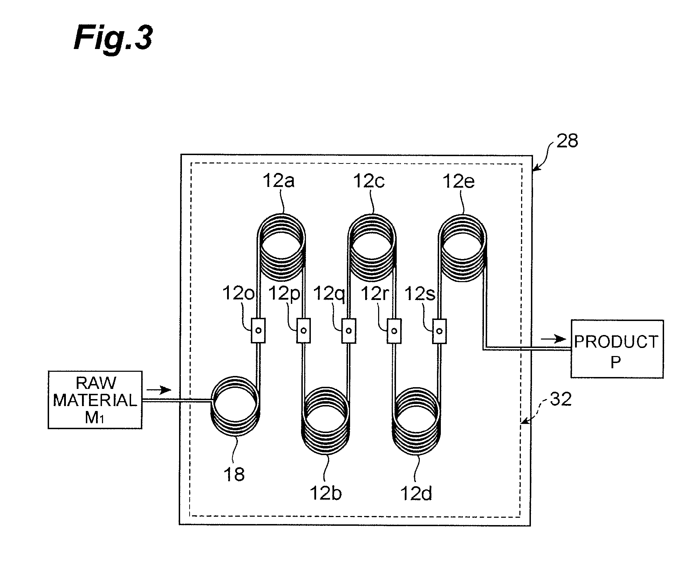

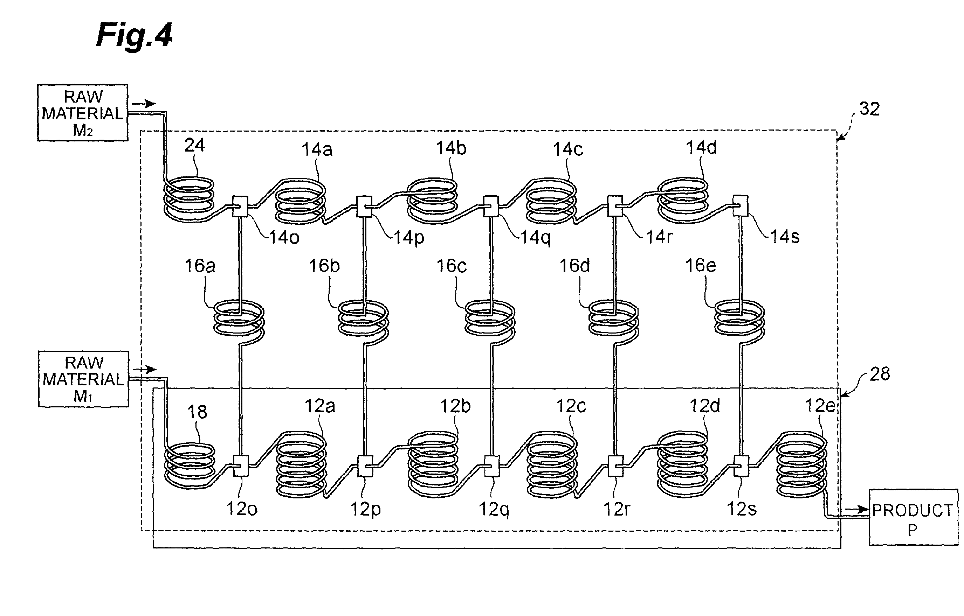

Figure 3 illustrates a view of one example, which is viewed from the upper side, and Figure 4 illustrates a view which is viewed from the side face. As is illustrated above, it is preferable to arrange the main flow channel 12 in a horizontal direction in the thermostatic liquid tank 28, and arrange the branch introduction flow channels 16a to 16e in a perpendicular (vertical) direction. This is because the arrangement space is made as small as possible.

Subsequently, the operation of the above described reaction apparatus 10 will now be described below. A raw material M₁ is supplied to a main flow channel 12 by a pump 20. The temperature of the raw material M₁ to be supplied to the main flow channel 12 is controlled by an adjusting flow channel 18. On the other hand, a raw material M₂ is supplied to an introduction flow channel 14 by a pump 26. The temperature of the raw material M₂ to be supplied to the introduction flow channel 14 is controlled by an adjusting flow channel 24.

One part of the raw material M₂ is branched from the introduction flow channel 14 at a branch point of each of branch introduction flow channels 16a to 16e in the introduction flow channel 14, and flows to each of the branch introduction flow channels 16a to 16e. The raw material M₂ flowing through each of the branch introduction flow channels 16a to 16e is introduced to the main flow channel 12 at each of introduction points 12o to 12s. The raw material M₂ which has been introduced to the main flow channel 12 reacts with the raw material M₁ which has been flowing through the main flow channel 12. This reaction raises the temperature of the fluid. The raised temperature is cooled by the refrigerant in the thermostatic liquid tank 28. A produced liquid P which has been produced through the reaction is collected in a container 22.

In this reaction apparatus 10, the raw material M₂ is introduced into the main flow channel 12 at five introduction points 12o to 12s of the main flow channel 12, so that reaction apparatus 10 can react the raw material M₁ with the raw material M₂ in a distributed way. Thereby, the reaction apparatus can inhibit the temperature rise of the fluid due to the introduction of the raw material M₂ at one of introduction points 12o to 12s. In this reaction apparatus 10, a temperature rise at an introduction point is lower as the introduction points 12o to 12s is located downstream in a flow direction of the main flow channel 12, but the reaction apparatus 10 has a structure of making the flow channel length of the flow channel 12a between the introduction points 12o and 12p in the main flow channel 12, in which the temperature rise is the highest, longer than the flow channel lengths between other introduction points 12b to 12e, and accordingly can appropriately remove the heat. From these descriptions, the reaction apparatus 10 according to the present exemplary embodiment can provide high heat removal efficiency, and accordingly can provide a high yield of a target substance.

The reaction apparatus 10 according to the present exemplary embodiment has a larger cross-section area of a flow channel than a microreactor, and accordingly can prevent the plug-up of the flow channel and can secure the high productivity.

The reaction apparatus in the present embodiment can particularly show an effect, when a nucleophilic organometallic compound such as a Grignard reagent is used as any one of a raw material M₁ and a raw material M₂. In a microreactor, the temperature of the Grignard reagent approaches that of a refrigerant in a flow channel because the fluid is effectively cooled, and a speed of the fluid in the flow channel is low, so that the plug-up of the flow channel tends to occur due to precipitates, and a refrigerant cannot employ a temperature not higher than a precipitation temperature as its temperature. However, in the present embodiment, the refrigerant can employ a temperature not higher than the precipitation temperature as its temperature. Accordingly, the reaction apparatus can keep cooling efficiency high, simultaneously can cool a reactive fluid in a main flow channel 12 to a low temperature to which the microreactor cannot cool the reactive fluid, and accordingly contribute to the improvement of the yield of a target substance.

In addition, the reaction apparatus can very effectively remove the heat, accordingly does not need to dilute the raw materials M₁ and M₂ with an organic solvent, and can save the organic solvent.

Specific examples of a Grignard reagent include methyl magnesium bromide, ethyl magnesium bromide, propyl magnesium bromide, allyl magnesium bromide, phenyl magnesium bromide, methyl magnesium chloride, ethyl magnesium chloride, propyl magnesium chloride, allyl magnesium chloride and phenyl magnesium chloride.

A specific example in the case of employing the Grignard reagent includes the following reaction. In this example, a Grignard reagent represented by the following formula (2) adds to a carbonyl compound represented by the following formula (1), and then, an adduct represented by the following formula (3) is obtained through two steps. In the formula, Ar¹ represents an aryl group.

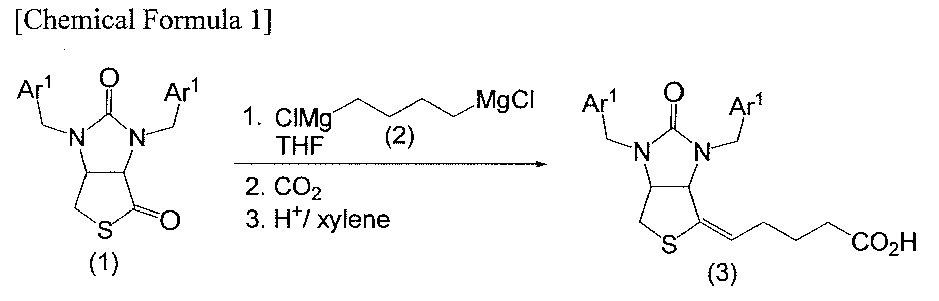

Another specific example of using the Grignard reagent includes a reaction through which an adduct represented by the following formula (8) is obtained when allyl magnesium chloride represented by the following formula (7) adds to an arylaldehyde. In the formula, Ar² represents an aryl group, and Ar³ represents an arylene group.

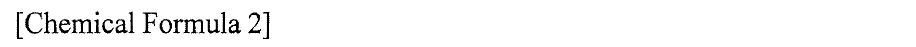

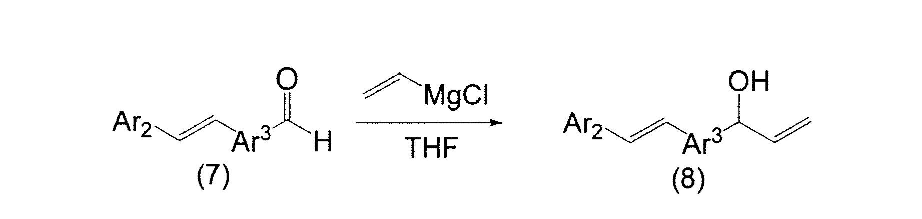

A reaction other than the Grignard reagent can include an addition reaction of the nucleophilic organometallic compound with an organocyano compound or a carbonyl compound by using an organolithium compound such as a lithium alkyl compound, an organozinc compound such as a zinc alkyl, an organocadmium compound such as a cadmium dialkyl or an organosodium compound such as a sodium alkyl, as one of the raw materials M₁ and M₂, and using an organocyano compound or a carbonyl compound as the other of the raw materials M₁ and M₂.

The above described organolithium compound includes lithium methyl, lithium butyl and lithium phenyl. A carbonyl compound includes a compound having a functional group such as an alkylcarbonyl group, an arylcarbonyl group, an alkoxycarbonyl group, an aryloxycarbonyl group and an amino-carbonyl group, and specifically, an acyl group, a methoxycarbonyl group, an ethoxycarbonyl group, a propyloxycarbonyl group, an amino-carbonyl group and a dimethylamino-carbonyl group.

A specific example in the case of using an organolithium compound includes the following reaction. In this example, the reaction is caused by passing n-butyl lithium and diethylamine through the first reaction apparatus (its introduction flow channel) according to the present invention as a second fluid, and passing ethylacetoacetate represented by the following formula (4) as a first fluid (through main flow channel); and by passing the above product through the second reaction apparatus (its introduction flow channel) according to the present invention as a second fluid, and passing 1-aryl-3-hexanon (through main flow channel) as a first fluid. Thereby, the reaction apparatus according to the present invention can be applied to a method of obtaining 5,5-dihydro-4-hydroxy-6-(arylethyl)-6-propyl-2, H-pyran-2-on represented by the following formula (6). In the formula, Ar⁴ represents an aryl group.

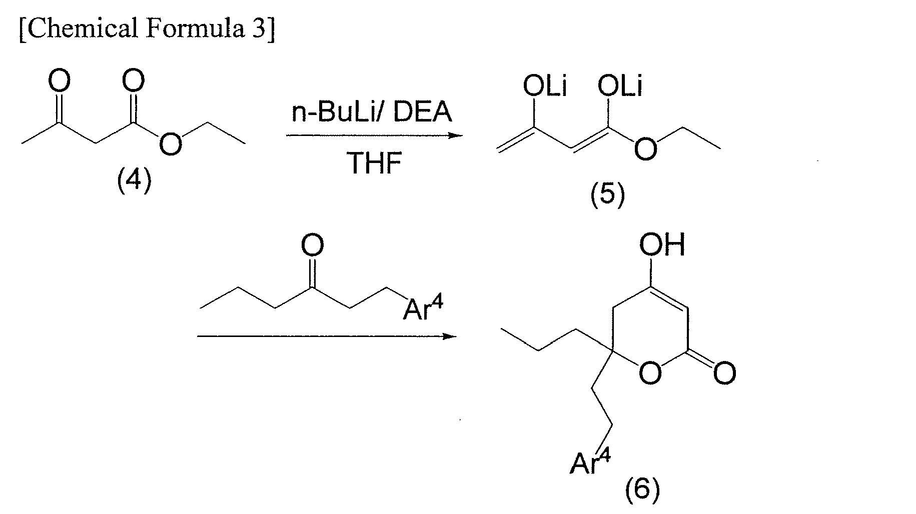

This method can obtain a high total yield, by obtaining a lithium compound which is a product to be obtained in the first reaction apparatus according to the present invention at the maximum yield, and continuously passing a carbonyl compound to an apparatus to react the compounds with each other, as was described above.

When the lithium compound (5) is held for a period of time, it continuously disappears due to a side reaction, so that it shows a large effect in enhancing the total yield to connect the reaction apparatuses according to the present invention and passing the following reactant without holding the product in the first reaction apparatus for a period of time, as was described above.

An exchange reaction of an active hydrogen with a metal such as lithium or sodium can be carried out by employing an organolithium compound such as a lithium alkyl compound or an organosodium compound such as a sodium alkyl, as one of the raw materials M₁ and M₂, and employing a compound having an active hydrogen such as a secondary amine as the other of the raw materials M₁ and M₂. For instance, lithium diisopropylamide is obtained by employing butyl lithium and diisopropylamine as the raw materials M₁ and M₂.

Furthermore, an exchange reaction of a halogen atom with a metal such as lithium or sodium can be carried out by employing an organolithium compound such as a lithium alkyl compound or an organosodium compound such as a sodium alkyl, as one of the raw materials M₁ and M₂, and employing an organic compound having a halogen atom as the other of the raw materials M₁ and M₂.

Still furthermore, a reduction reaction of a carbonyl compound can be carried out by employing a metal hydride such as lithium aluminum hydride, lithium borohydride and sodium borohydride, a metal or an alloy as one of the raw materials M₁ and M₂, and employing a carbonyl compound as the other of the raw materials M₁ and M₂.

In addition to the above description, a hydrogenation of an unsaturated compound can be carried out by employing a metal complex having a ferroceno phosphine as a ligand as one of the raw materials M₁ and M₂, and employing an unsaturated compound as the other of the raw materials M₁ and M₂.

The ferroceno phosphine ligand which can be applied to this case includes:
Josiphos ligand represented by the following chemical formula (wherein R and R' represent an organic group); 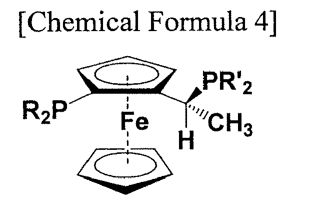 Walphos ligand represented by the following chemical formula (wherein R and R' represent an organic group); and 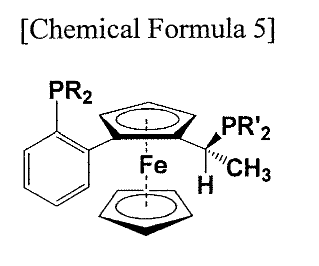 Mandyphos ligand represented by the following chemical formula (wherein R and R' represent an organic group).

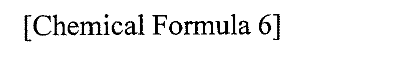

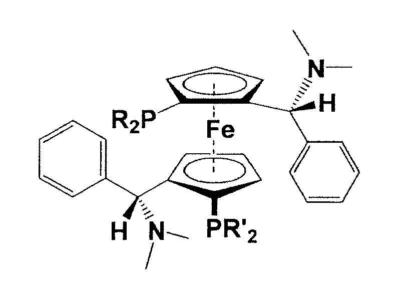

The metallic complex having the ferroceno phosphine as a ligand is particularly preferably a rhodium complex having the ligand as described above.

A specific example of a hydrogenation reaction with the use of a rhodium complex includes a chiral hydrogenation reaction (catalytic reaction with the use of chiral uniform rhodium complex) expressed by the following reaction formula. In the following formula, Rh-COD represents rhodium-cyclooctadienyl; R represents a C₁ to C₆ alkyl group which may be substituted with a halogen atom; Ar represents an aryl group (which may be substituted with a C₁ to C₆ alkyl group optionally substituted with a halogen atom, or a halogen atom); and Josiphos represents the above described Josiphos ligand (R represents a 4-trifluoromethylphenyl group and R' represents a t-butyl group, or R represents a phenyl group and R' represents a t-butyl group).

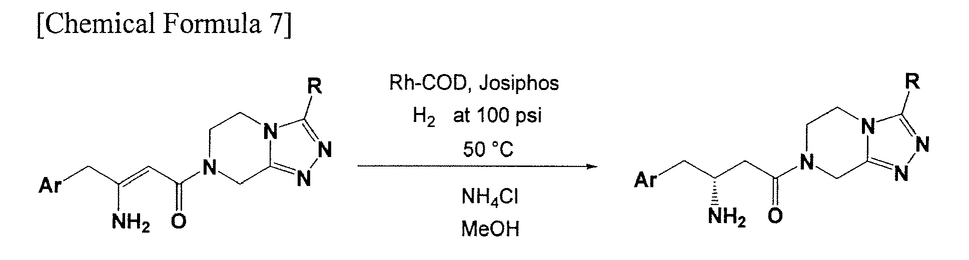

A reaction apparatus 10 according to the present embodiment shown in the above described Figure 1 makes all flowing-type fme reaction flow channels 32 arranged in the thermostatic liquid tank 28, but may not make all of them arranged in the thermostatic liquid tank 28. For instance, similarly to the reaction apparatus 50 shown in Figure 2, the reaction apparatus may have a structure in which only the main flow channel 12, a part close to the main flow channel 12 out of the branch introduction flow channels 16a to 16e, and the adjusting flow channel 18 are arranged in the thermostatic liquid tank 28. This structure can be applied to the cases in which the temperature of the raw material M₂ is sufficiently controlled by controlling the temperature in the part only close to the main flow channel 12 out of the branch introduction flow channels 16a to 16e.

Specifically, a portion from the main flow channel 12 to 5 cm, for instance, out of the branch introduction flow channels 16a to 16e is arranged in the thermostatic liquid tank 28.

### Example 1

The present invention will now be described more specifically below with reference to examples, but the present invention is not limited to the following examples.

### (Example 1)

Example 1 was carried out in a reaction apparatus 10 shown in Figure 1. A main flow channel 12 is a circular pipe made from stainless steel, and introduction points 12o and 12p are 180-degree T-shaped mixture flow channels made from stainless steel. A Grignard reagent: 1-bromomagnesium-5-chloropentane (0.45 mol/L) was diluted by a tetrahydrofuran solvent and the diluted solution was used as a raw material M₂. The reagent 1-bromomagnesium-5-chloropentane was prepared by adding a magnesium powder to 1-bromo-5-chloropentane. Diethyl oxalate (7.4 mol/L) which had not been diluted by the solvent was used as a raw material M₁. The raw material M₂ and the raw material M₁ were supplied to a flowing-type fine reaction flow channel 32, at 110 mL/min and 5.1 mL/min, respectively. Used pumps 20 and 26 were a smooth flow pump made by TACMINA CORPORATION. From the above described conditions, a mixed solution of these materials stays in the flowing-type fine reaction flow channel 32 for approximately 14 seconds.

The Grignard reagent and diethyl oxalate were accommodated in a supply container and controlled at 10°C and room temperature respectively, and a thermostatic liquid tank 28 accommodated methanol as a refrigerant 30 and was controlled at -15°C. The produced liquid was collected and quenched with a dilute hydrochloric acid. The target substance of ethyl-7-chloro-2-oxalic pentane was obtained in the yield of 90%.

### (Example 2)

Example 2 was carried out in a reaction apparatus 50 shown in Figure 2. Here, each length of branch introduction flow channels 16a to 16e was set at 0.5 m, and a portion from a main flow channel 12 to 5 cm of branch introduction flow channels 16a to 16e was arranged in a thermostatic liquid tank 28. The length of an adjusting flow channel 18 was set at 1 m. Each of the flow channel lengths of a flow channel 12a between introduction points 12o and 12p in the main flow channel 12 and a flow channel 12b between introduction points 12p and 12q was set at 3 m, and each of the flow channel lengths of a flow channel 12c between introduction points 12q and 12r, a flow channel 12d between introduction points 12r and 12s and a flow channel 12e from an introduction point 12s to an edge of the thermostatic liquid tank 28 was set at 1 m. The other conditions were set at the same condition as in Example 1.

A Grignard reagent: 1-bromomagnesium-5-chloropentane (0.45 mol/L) was diluted by a tetrahydrofuran solvent and the diluted solution was used as a raw material M₂. Diethyl oxalate (7.4 mol/L) which had not been diluted by the solvent was used as a raw material M₁. The raw material M₂ and the raw material M₁ were supplied to a flowing-type fine reaction flow channel 32, at 110 mL/min and 5.1 mL/min, respectively. From the above described conditions, a mixed solution of these materials stays in the flowing-type fine reaction flow channel 32 for approximately 42 seconds.

The Grignard reagent and diethyl oxalate were accommodated in a supply container and controlled at 10°C and room temperature respectively, and the thermostatic liquid tank 28 accommodated methanol as a refrigerant 30 and was controlled at -15°C. The produced liquid was collected and quenched with a dilute hydrochloric acid. The target substance of ethyl-7-chloro-2-oxalic pentane was obtained in the yield of 90%.

### (Example 2')

Example 2' was carried out while controlling the temperature of a thermostatic liquid tank 28 to -5°C, and setting other conditions at the same conditions as in Example 2. As a result, the yield of ethyl-7-chloro-2oxalic pentane was 86%.

### (Example 3)

Example 3 was carried out in a reaction apparatus 50 shown in Figure 2. Here, a circular pipe having an inner diameter 3 mm and an outer diameter 4 mm was used for the pipe which constitutes a main flow channel 12, an introduction flow channel 14 and each of branch introduction flow channels 16a to 16e. The lengths of the respective branch introduction flow channels 16a to 16e were set at 0.5 m. The length of an adjusting flow channel 18 was set at 1 m. The flow channel lengths of a flow channel 12a between introduction points 12o and 12p in the main flow channel 12 and a flow channel 12b between introduction points 12p and 12q were each set at 3 m, and the flow channel lengths of a flow channel 12c between introduction points 12q and 12r, a flow channel 12d between introduction points 12r and 12s and a flow channel 12e from an introduction point 12s to an edge of a thermostatic liquid tank 28 were set at 1 m respectively. The other conditions were the same as in Example 1.

A Grignard reagent: 1-bromomagnesium-5-chloropentane (0.45 mol/L) was diluted by a tetrahydrofuran solvent and the diluted solution was used as a raw material M₂. Diethyl oxalate (7.4 mol/L) which had not been diluted by the solvent was used as a raw material M₁. The raw material M₂ and the raw material M₁ were supplied to a flowing-type fine reaction flow channel 32, at 965 mL/min and 47 mL/min, respectively. From the above described conditions, a mixed solution of these materials stays in a flowing-type fine reaction flow channel 32 for approximately 4.9 seconds.

The Grignard reagent and diethyl oxalate were accommodated in a supply container and controlled at 10°C and room temperature respectively, and the thermostatic liquid tank 28 accommodated methanol as a refrigerant 30 and was controlled at -30°C. The produced liquid was collected and quenched with a dilute hydrochloric acid. The target substance of ethyl-7-chloro-2-oxalic pentane was obtained in the yield of 88%.

### (Example 4)

Example 4 was carried out in a reaction apparatus 50 shown in Figure 2. Here, a circular pipe having an inner diameter 1 mm and an outer diameter 3 mm was used for the pipe which constitutes a main flow channel 12. A circular pipe having an inner diameter 3 mm and an outer diameter 4 mm was used for the pipe which constitutes an introduction flow channel 14. A circular pipe having an inner diameter 1 mm and an outer diameter 3 mm was used for the pipe which constitutes each of branch introduction flow channels 16a to 16e. Lengths of branch introduction flow channels 16a to 16e were set at 0.5 m, 1.5 m, 2.0 m, 2.5 m and 3.0 m respectively, and portions from the main flow channel 12 to 5 cm of all branch introduction flow channels were arranged in a thermostatic liquid tank 28. The length of an adjusting flow channel 18 was set at 1 m. The flow channel length of a flow channel 12a between introduction points 12o and 12p in the main flow channel 12 was set at 1.0 m, and the flow channel lengths of a flow channel 12b between introduction points 12p and 12q, a flow channel 12c between introduction points 12q and 12r, a flow channel 12d between introduction points 12r and 12s, and a flow channel 12e from an introduction point 12s to an edge of the thermostatic liquid tank 28 were set at 0.5 m respectively. The other conditions were the same as in the above described reaction apparatus 10.

A Grignard reagent: 1-bromomagnesium-5-chloropentane (0.45 mol/L) was diluted by a tetrahydrofuran solvent and the diluted solution was used as a raw material M₂. Diethyl oxalate (7.4 mol/L) which had not been diluted by the solvent was used as a raw material M₁. The raw material M₂ and the raw material M₁ were supplied to a flowing-type fine reaction flow channel 32, at 109 mL/min and 5.1 mL/min, respectively. From the above described conditions, a mixed solution of these materials stays in the flowing-type fine reaction flow channel 32 for approximately 1.6 seconds.

The Grignard reagent and diethyl oxalate were accommodated in a supply container and controlled at 10°C and room temperature respectively, and the thermostatic liquid tank 28 accommodated methanol as a refrigerant 30 and was controlled at -15°C. The produced liquid was collected and quenched with a dilute hydrochloric acid. The target substance of ethyl-7-chloro-2-oxalic pentane was obtained in the yield of 90%.

### (Example 4')

Example 4' was carried out while controlling the temperature of a thermostatic liquid tank 28 to 5°C, and setting other conditions at the same conditions as in Example 4. As a result, the yield of ethyl-7-chloro-2oxalic pentane was 89%.

### (Example 5)

Example 5 was carried out in a reaction apparatus 50 shown in Figure 2. Here, a circular pipe having an inner diameter 1 mm and an outer diameter 3 mm was used for the pipe which constitutes a main flow channel 12. A circular pipe having an inner diameter 3 mm and an outer diameter 4 mm was used for the pipe which constitutes an introduction flow channel 14. A circular pipe having an inner diameter 1 mm and an outer diameter 3 mm was used for the pipe which constitutes each of branch introduction flow channels 16a to 16e. Each length of branch introduction flow channels 16a to 16e was set at 0.5 m, and portions from the main flow channel 12 to 5 cm of all the branch introduction flow channels 16a to 16e were arranged in a thermostatic liquid tank 28. The length of an adjusting flow channel 18 was set at 1 m. The flow channel length of a flow channel 12a between introduction points 12o and 12p in the main flow channel 12 was set at 1.0 m, and the flow channel lengths of a flow channel 12b, a flow channel 12c, a flow channel 12d, and a flow channel 12e respectively between introduction points 12p and 12q, between introduction points 12q and 12r, between introduction points 12r and 12s, and from an introduction point 12s to an edge of the thermostatic liquid tank 28 were set at 0.5 m. The other conditions were the same as in the above described reaction apparatus 10.

A Grignard reagent: 1-bromomagnesium-5-chloropentane (0.45 mol/L) was diluted by a tetrahydrofuran solvent and the diluted solution was used as a raw material M₂. Diethyl oxalate (2.0 mol/L) which had been diluted by the same solvent was used as a raw material M₁. The raw material M₂ and the raw material M₁ were supplied to a flowing-type fine reaction flow channel 32, at 100 mL/min and 17 mL/min, respectively. From the above described conditions, a mixed solution of these materials stays in the flowing-type fine reaction flow channel 32 for approximately 1.4 seconds.

The Grignard reagent and diethyl oxalate were accommodated in a supply container and controlled at 10°C and room temperature respectively, and the thermostatic liquid tank 28 accommodated methanol as a refrigerant 30 and was controlled at -15°C. The produced liquid was collected and quenched with a dilute hydrochloric acid. The target substance of ethyl-7-chloro-2-oxalic pentane was obtained in the yield of 84%.

### (Comparative example 1)

The above described reaction was carried out by using a microreactor system made by Cellular Process Chemistry GmbH. The Grignard reagent: 1-bromomagnesium-5-chloropentane (0.45 mol/L) was diluted by a tetrahydrofuran solvent and the diluted solution was used as a raw material M₂. Diethyl oxalate (5.5 mol/L) which had been diluted by the same solvent was used as a raw material M₁. The raw material M₂ and the raw material M₁ were supplied to a flowing-type fine reaction flow channel, at 16 mL/min and 1 mL/min, respectively. The reaction apparatus was kept at -5°C by an attached temperature controller. The yield of a target substance was 84%, but plug-up occurred and the raw materials could not be supplied on the way.

### (Comparative example 2)

In comparison with Example 3, branch introduction flow channels 16a to 16e to be connected to the main flow channel 12 were made to be one channel instead of 5 channels. Comparative example 2 was carried out under the same conditions as in Example 3, except the above described condition. The yield of a target substance was 74%.

### (Result)

The result of the above described examples will now be described together in the following Table.

**[Table 1]**

| | Example 1 | Example 2 | Example 2' | Example 3 | Example 4 | Example 4' | Example 5 |
|---|---|---|---|---|---|---|---|
| reaction apparatus | reaction apparatus 10 | reaction apparatus 50 | reaction apparatus 50 | reaction apparatus 50 | reaction apparatus 50 | reaction apparatus 50 | reaction apparatus 50 |
| pipe of main flow channel 12 | outer diameter 4 mm inner diameter 3 mm | outer diameter 4 mm inner diameter 3 mm | outer diameter 4 mm inner diameter 3 mm | outer diameter 4 mm inner diameter 3 mm | outer diameter 3 mm inner diameter 1 mm | outer diameter 3 mm inner diameter 1 mm | outer diameter 3 mm inner diameter 1 mm |
| 12a | 1.0 m | 3.0 m | 3.0 m | 3.0 m | 1.0 m | 1.0 m | 1.0 m |
| 12b | 0.5 m | 3.0 m | 3.0 m | 3.0 m | 0.5 m | 0.5 m | 0.5 m |
| 12c | 0.5 m | 1.0 m | 1.0 m | 1.0 m | 0.5 m | 0.5 m | 0.5 m |
| 12d | 0.5 m | 1.0 m | 1.0 m | 1.0 m | 0.5 m | 0.5 m | 0.5 m |
| 12e | 0.5 m | 1.0 m | 1.0 m | 1.0 m | 0.5 m | 0.5 m | 0.5 m |
| pipe of introduction flow channel 14 | outer diameter 4 mm inner diameter 3 mm | outer diameter 4 mm inner diameter 3 mm | outer diameter 4 mm inner diameter 3 mm | outer diameter 4 mm inner diameter 3 mm | outer diameter 4 mm inner diameter 3 mm | outer diameter 4 mm inner diameter 3 mm | outer diameter 4 mm inner diameter 3 mm |
| 14a | 1.0 m | 0.5 m | 0.5 m | 1.0 m | 0.5 m | 0.5 m | 0.5 m |
| 14b | 0.5 m | 0.5 m | 0.5 m | 1.0 m | 0.5 m | 0.5 m | 0.5 m |
| 14c | 0.5 m | 0.5 m | 0.5 m | 1.0 m | 0.5 m | 0.5 m | 0.5 m |
| 14d | 0.5 m | 0.5 m | 0.5 m | 1.0 m | 0.5 m | 0.5 m | 0.5 m |
| pipe of branch introduction flow channel 16 | outer diameter 3 mm inner diameter 1 mm | outer diameter 3 mm inner diameter 1 mm | outer diameter 3 mm inner diameter 1 mm | outer diameter 4 mm inner diameter 3 mm | outer diameter 3 mm inner diameter 1 mm | outer diameter 3 mm inner diameter 1 mm | outer diameter 3 mm inner diameter 1 mm |
| 16a | 0.5 m | 0.5 m | 0.5 m | 0.5 m | 0.5 m | 0.5 m | 0.5 m |
| 16b | 0.5 m | 0.5 m | 0.5 m | 0.5 m | 1.5m | 1.5m | 0.5 m |
| 16c | 0.5 m | 0.5 m | 0.5 m | 0.5 m | 2.0 m | 2.0 m | 0.5 m |
| 16d | 0.5 m | 0.5 m | 0.5 m | 0.5 m | 2.5 m | 2.5 m | 0.5 m |
| 16e | 0.5 m | 0.5 m | 0.5 m | 0.5 m | 3.0 m | 3.0 m | 0.5 m |
| arrangement length of thermostatic liquid tank 28 of branch introduction flow channel 16 | 0.5 m | 5 cm | 5 cm | 5 cm | 5 cm | 5 cm | 5 cm |
| thermostatic liquid tank 28 | -15°C | -15°C | -5°C | -30°C | -15°C | 5°C | -15°C |
| yield | 90% | 90% | 86% | 88% | 90% | 89% | 84% |

The reactions according to the above described examples are an exothermic reaction (reaction heat: approximately 100 kJ/mol), so that it becomes necessary for inhibiting the production of a reaction product and effectively producing a target substance to remove a heat which has been generated through the reaction with high efficiency, and to keep the temperature in a main flow channel 12 which is a reaction flow channel at as low a temperature as possible. The equivalent diameter of the main flow channel 12 in the examples is 1 to 3 mm, which can inhibit the temperature from rising. Furthermore, the reaction apparatus of the present invention adopts a structure which is formed of a main flow channel, an introduction flow channel and a branch introduction flow channel having simple and easy flow channel diameters and lengths, and a system which has a plurality of branch introduction flow channels, and thereby can distribute the reaction heat to be generated at an introduction point and can remove the heat from the reaction fluid more effectively so as to set the temperature in a flow channel at a predetermined temperature.

In addition, reaction apparatuses 10 and 50 according to the present examples could provide a higher reaction yield than a microreactor system. Furthermore, the flow channel of the microreactor system was very small, and frequently caused plug-up therein. However, the reaction apparatuses 10 and 50 according to the present examples had flow channels with diameters of 1 to 3 mm, and accordingly did not cause the plug-up at all. The microreactor system is largely affected by a pressure loss in the flow channel, and accordingly could not send a large amount of flow rates. However, the reaction apparatuses 10 and 50 according to the present examples are less affected by the pressure loss, can send a large amount of flow rates, accordingly can be stably operated continuously for a long period of time, and is suitable for a high-volume production.

## Claims

1. A reaction apparatus having a main flow channel which has a cross-section area corresponding to an area of a circle having a diameter of 0.5 to 6 mm and in which a first fluid flows,
an introduction flow channel in which a second fluid that causes a chemical reaction with the first fluid flows, and
three or more branch introduction flow channels which are branched from the introduction flow channel and introduce the second fluid to the main flow channel at predetermined introduction points in the main flow channel, **characterized in that**
a flow channel length between adjacent introduction points in the main flow channel is not longer than a flow channel length between next previous adjacent introduction points in a flow direction of the first fluid, and at least one flow channel length between the adjacent introduction points is shorter than flow channel lengths between previous adjacent introduction points in the flow direction of the first fluid.

2. The reaction apparatus according to claim 1, **characterized in that** the reaction apparatus further has temperature control means for controlling the temperature of the main flow channel and the vicinity of the introduction point in the branch introduction flow channel.

3. The reaction apparatus according to claim 1 or 2, **characterized in that** the cross-section area of the main flow channel is equivalent to the area of a circle having a diameter of 1 to 3 mm.

4. The reaction apparatus according to any one of claims 1 to 3, **characterized in that**
the introduction point is structured by a 180-degree T-shaped mixture channel, and
the branch introduction flow channel is perpendicularly connected to the main flow channel.

5. The reaction apparatus according to any one of claims 1 to 4, **characterized in that** the number of the branch introduction flow channels is 5 to 10.

6. The reaction apparatus according to any one of claims 1 to 5, **characterized in that**
the reaction apparatus further has a first adjusting flow channel for adjusting the temperature of the first fluid before the first fluid is supplied to the main flow channel, and
a second adjusting flow channel for adjusting the temperature of the second fluid before the second fluid is supplied to the introduction flow channel.

7. The reaction apparatus according to any one of claims 1 to 6, **characterized in that**
the main flow channel, the introduction flow channel and the branch introduction flow channel have the same cross-section area, and
the branch introduction flow channels have the same flow channel length.

8. The reaction apparatus according to any one of claims 1 to 6, **characterized in that**
the main flow channel and the branch introduction flow channel have the cross-section areas not larger than that of the introduction flow channel, and
the branch introduction flow channels have the same flow channel length.

9. The reaction apparatus according to any one of claims 1 to 6, **characterized in that**
the main flow channel and the branch introduction flow channel have the cross-section areas not larger than that of the introduction flow channel, and
the branch introduction flow channel has a flow channel length not shorter than that of a branch introduction flow channel to be connected to the main flow channel at the introduction point next previous to the introduction point of the branch introduction flow channel, in the flow direction of the main flow channel.

10. The reaction apparatus according to any one of claims 1 to 9, **characterized in that**
the reaction apparatus further has a first pump which supplies the first fluid to the main flow channel, and a second pump which supplies the second fluid to the introduction flow channel.

11. The reaction apparatus according to claim 10, **characterized in that** the first pump and the second pump are a double diaphragm pump which employs a non-circular cam therein.

12. A reaction method using the reaction apparatus according to any one of claims 1 to 11, **characterized in that**
the reaction method passes a fluid containing a nucleophilic organometallic compound as one of the first fluid and the second fluid, and passes a fluid containing a compound which causes an addition reaction or an exchange reaction with the nucleophilic organometallic compound, as the other fluid.

13. The reaction method according to claim 12, **characterized in that** the reaction method passes the fluid containing the nucleophilic organometallic compound, as the second fluid, and passes the fluid containing the compound which causes the addition reaction or the exchange reaction with the nucleophilic organometallic compound, as the first fluid.

14. The reaction method according to claim 12, **characterized in that** the first fluid and the second fluid contain at least one type of solvent selected from the group consisting of tetrahydrofuran, diethyl ether, dioxane and dibutyl ether.

15. The reaction method according to claim 12, **characterized in that** the fluid containing the nucleophilic organometallic compound out of the first fluid and the second fluid contains at least one type of solvent selected from the group consisting of tetrahydrofuran, diethyl ether, dioxane and dibutyl ether, and the fluid containing the compound which causes the addition reaction or the exchange reaction with the nucleophilic organometallic compound does not contain a solvent.

16. The reaction method according to any one of claims 12 to 15, **characterized in that** the nucleophilic organometallic compound is at least one type of nucleophilic organometallic compound selected from the group consisting of an organomagnesium compound, an organolithium compound, an organozinc compound, an organocadmium compound and an organosodium compound.

17. The reaction method according to claim 16, **characterized in that** the organomagnesium compound is a Grignard reagent.

18. The reaction method according to any one of claims 12 to 17, **characterized in that** the compound which causes the addition reaction or the exchange reaction with the nucleophilic organometallic compound is a carbonyl compound.

19. The reaction method according to any one of claims 12 to 15, **characterized in that**
the nucleophilic organometallic compound is 1-bromomagnesium-5-chloropentane, and
the compound which causes the addition reaction or the exchange reaction with the nucleophilic organometallic compound is diethyl oxalate.

20. A reaction method using the reaction apparatus according to any one of claims 1 to 11, **characterized in that**
the reaction method passes a fluid containing a catalyzer for a reaction selected from hydrogenation or reduction and hydrogen, as one of the first fluid and the second fluid, and passes a fluid containing a substrate for the reaction, as the other.

21. The reaction method according to claim 20, **characterized in that** the reaction method passes the fluid containing the catalyzer which is made for a hydrogenation reaction from a metallic complex having ferroceno phosphine as a ligand, and hydrogen, as one of the first fluid and the second fluid, and passes a fluid containing an unsaturated compound, as the other.

22. The reaction method according to claim 21, **characterized in that** the metal complex is a rhodium complex having ferroceno phosphine as a ligand.
